# EUROPÄISCHE PATENTSCHRIFT

(11) **EP 2 397 177 B1**
(45) Veröffentlichungstag und Bekanntmachung des Hinweises auf die Patenterteilung: **29.06.2016**
(21) Anmeldenummer: 11166167.4
(22) Anmeldetag: 16.05.2011
(51) Int. Cl.: B05B 17/06, A61M 11/00, A61M 15/00, B05B 17/00

(54) **Zerstäubervorrichtung**
Atomiser device
Dispositif d'atomisation

(30) Priorität: 15.06.2010 DE 102010024913
(43) Veröffentlichungstag der Anmeldung: 21.12.2011
(73) Patentinhaber: Aptar Radolfzell GmbH, 78315 Radolfzell (DE)
(72) Erfinder: Kohnle, Jörg, 78056, VS-Schwenningen (DE); Keppner, Frank, 72458, Albstadt (DE); Pfeffer, Hubertus, 78357, Schwackenreute (DE)
(74) Vertreter: Patentanwaltskanzlei Cartagena

(56) Entgegenhaltungen:
- DE-A1-102004 006 452
- DE-A1-102004 011 726
- DE-A1-102006 061 506
- DE-T2- 60 120 614
- JP-A- S6 054 761
- JP-A- S6 197 066
- US-A1- 2010 071 687

## Beschreibung

Die Erfindung betrifft einen Zerstäuber zur Zerstäubung von Flüssigkeiten, insbesondere von pharmazeutischen oder kosmetischen Flüssigkeiten. Ein gattungsgemäßer Zerstäuber umfasst einen Flüssigkeitsspeicher sowie eine Zerstäubungseinrichtung mit einer Zerstäubungskammer, die durch eine Vielzahl von Auslassöffnungen mit einer Umgebungsatmosphäre verbunden ist, und mit einer Vibrationseinrichtung, mittels derer zum Zwecke der Zerstäubung Flüssigkeit in der Zerstäubungskammer in Schwingungen versetzbar ist. Weiterhin umfasst ein gattungsgemäßer Zerstäuber einen Zulaufkanal, durch den der Flüssigkeitsspeicher mit der Zerstäubungseinrichtung verbunden ist, und eine Pumpeinrichtung, die im Zulaufkanal zum Zwecke der Förderung der Flüssigkeit vom Flüssigkeitsspeicher zur Zerstäubungskammer vorgesehen ist. Zur Steuerung der Pumpeinrichtung und der Vibrationseinrichtung ist eine elektronische Steuereinrichtung vorgesehen.

Gattungsgemäße Zerstäuber sind aus dem Stand der Technik bekannt. Ihr Verwendungszweck liegt darin, Flüssigkeiten zu einem feinen Nebel zu zerstäuben. Bei diesen Flüssigkeiten handelt es sich insbesondere um pharmazeutische oder kosmetische Flüssigkeiten. Bei pharmazeutischen Flüssigkeiten kann der Zweck der Zerstäubung beispielsweise darin liegen, hierdurch einen besonders wirkungsvoll inhalierbaren Nebel zu erzeugen. Bei kosmetischen Flüssigkeiten kann der Zweck der Zerstäubung darin liegen, hierdurch einen besonders homogenen Austrag, beispielsweise zur Erzeugung einer besonders homogenen Schicht der Flüssigkeit auf einer Hautpartie, zu erzielen.

Aus DE 10 2004 011 726 A1 ist eine Dosiervorrichtung bekannt, welche einen Dosierraum aufweist, wobei Flüssigkeit aus einem Mediumspeicher in einen Ringkanal und einen Dosierraum gefördert wird und überschüssiges Medium während des Fördervorgangs durch Belüftungsöffnung zurück in den Mediumspeicher strömt. Nach einem Befüllen des Dosierraums werden der Dosierraum und der Ringkanals von dem Mediumspeicher getrennt und nach der Trennung erfolgt der Austrag der begrenzten Flüssigkeitsmenge. Die begrenzten Flüssigkeitsmenge ist vorzugsweise so bemessen, dass sie für eine Anzahl von Austragvorgängen ausreicht.

Aus US 2010/0071687, JP S61 97066 A und JP S60 54761 A sind Zerstäuber bekannt, die eine Rückführung von überschüssiger Flüssigkeit aus der Zerstäuberkammer in den Flüssigkeitsspeicher während der Zerstäubung zeigen.

Die Pumpeinrichtung gattungsgemäßer Zerstäuber kann dem Zweck dienen, die Zerstäubungseinrichtung kontinuierlich mit Flüssigkeit zu versorgen. Es hat sich jedoch herausgestellt, dass es schwierig ist, die Pumpeinrichtung und die Zerstäubungseinrichtung derart aufeinander abzustimmen, dass der von der Pumpeinrichtung zur Verfügung gestellte Pump-Volumenstrom perfekt zur zerstäubten Flüssigkeitsmenge passt. Bei einem zu großen Pump-Volumenstrom besteht die Gefahr, dass die Zerstäubungscharakteristik dadurch verschlechtert wird, dass die Flüssigkeit in nicht zerstäubter Form durch die Austragöffnungen hindurchgelangt und auf der abgewandten Seite der Austragöffnungen einen die Zerstäubung behindernden Flüssigkeitsfilm bildet. Bei zu geringer Größe des Pump-Volumenstroms ist eine gute Zerstäubungsqualität ebenfalls nicht zu erreichen, da sich vergleichsweise große Luftmengen in der Zerstäubungskammer sammeln und dem Austrag der Flüssigkeit in zerstäubter Form entgegenstehen. In besonders problematischer Form ergeben sich diese Nachteile bei einem Zerstäuber, der in variablen Stellungen benutzbar sein soll. Diese Stellungsvariabilität kann aus verschiedenen Gründen wünschenswert sein. So ist beispielsweise bei einem Zerstäuber zum Zwecke der Inhalation von Medikamenten wünschenswert, dass dieser vom Patienten in verschiedenen Ausrichtungen, beispielsweise sowohl stehend als auch liegend, verwendet werden kann. Bei einem Zerstäuber für kosmetische Produkte, die auf verschiedenen Hautpartien aufzubringen sind, ist es nur schwer möglich, dies bei stets gleichbleibender Ausrichtung des Zerstäubers zu tun. Durch die variable Ausrichtung eines gattungsgemäßen Zerstäubers wird das oben dargestellte Problem verschärft, da das Eindringen von Luft in die Zerstäuberkammer begünstigt sein kann und die Druckverhältnisse in der Zerstäuberkammer in höherem Maße von der Ausrichtung und Bewegung des Zerstäubers abhängen.

Um das Problem der Abstimmung zwischen der Pumpeinrichtung und der Zerstäubungseinrichtung zu umgehen, ist es aus dem Stand der Technik, beispielsweise aus der DE 10 2004 006 452 A1, bekannt, die Pumpeinrichtung und die Vibrationseinrichtung nacheinander zu aktivieren. Zunächst wird über die Pumpeinrichtung die Zerstäubungskammer selbst sowie ggf. ein damit in unmittelbarer Verbindung stehender Kapilarkanal gefüllt. Anschließend wird die Vibrationseinrichtung aktiviert, um die so in die Zerstäubungskammer und dem Kapilarkanal eingebrachte Flüssigkeitsmenge auszutragen. Gemäß DE 10 2004 006 452 A1 ist ein Dosierraum mit einem Mediumspeicher über einen ersten Zulaufkanal kommuniziert und weiter, um eine gleichmäßige Versorgung des Dosierraums während eines Austragvorgangs zu gewährleisten, eine mit dem Dosierraum über einen zweiten Zulaufkanal kommunizierte Vorratskammer vorgesehen, welche als temporärer Speicher für auszutragende Flüssigkeit dient. Nachteil einer solchen Gestaltung ist, dass die auszutragende Flüssigkeitsmenge gering ist. Ein kontinuierlicher Zerstäubungsvorgang ist hierdurch nicht erreichbar. Je nach Art des Medikaments, insbesondere jedoch auch bei kosmetischen Flüssigkeiten, ist dies ein erheblicher Nachteil.

### Aufgabe und Lösung

Aufgabe der Erfindung ist es, einen gattungsgemäßen Zerstäuber dahingehend weiterzubilden, dass dieser unter Vermeidung erheblichen Anpassungsaufwandes zwischen der Pumpeinrichtung und der Zerstäubungseinrichtung eine kontinuierliche Zerstäubung gestattet. Insbesondere ist ein Zerstäuber angestrebt, der sich für einen Zerstäubungsvorgang bei variabler Ausrichtung eignet.

Erfindungsgemäß wird dies durch einen Zerstäuber mit den Merkmalen des Anspruchs 1 erreicht.

Bei einer solchen Gestaltung ist zusätzlich zum Zulaufkanal, über den die Flüssigkeit vom Flüssigkeitsspeicher über die Pumpeinrichtung bis zur Zerstäubungskammer der Zerstäubungseinrichtung geleitet wird, ein weiterer Kanal vorgesehen, der von der Zerstäubungskammer zum Flüssigkeitsspeicher führt. Primäre Aufgabe dieses Rücklaufkanals ist es, überschüssige Flüssigkeit von der Zerstäubungskammer zurück in den Flüssigkeitsspeicher zu fördern. Dies erlaubt es, der Zerstäubungskammer ein Volumenstrom der Flüssigkeit zuzuführen, der signifikant über dem von der Zerstäubungseinrichtung zerstäubbaren AustragVolumenstrom liegt. Die überschüssige Flüssigkeitsmenge wird nicht durch die Auslassöffnungen ausgetragen, sondern gelangt über den Rücklaufkanal zurück in den Flüssigkeitsspeicher. Der Rücklaufkanal ist hierfür passend zu dimensionieren. Dieser Aufbau gestattet es, eine kontinuierliche Flüssigkeitszerstäubung zu gewährleisten, bei der stets ausreichend Flüssigkeit zum Zerstäuben bereitsteht, indem ein deutlich über einem Zerstäubungs-Volumenstrom liegender Volumenstrom der Zerstäuberkammer kontinuierlich zugeführt wird. Des Weiteren ist selbst für jenen Fall, dass Luft durch die Auslassöffnungen hindurch in die Zerstäubungskammer gelangt, durch den Rücklaufkanal erreichbar, dass diese Luft aus der Zerstäubungskammer abgeführt wird und gemeinsam mit der überschüssigen Flüssigkeit in den Flüssigkeitsspeicher gelangt. Der erfindungsgemäße Zerstäuber gewährleistet so eine sehr reproduzierbaren und gleichbleibenden Zerstäubungsvorgang.

Die Zerstäubungseinrichtung eines erfindungsgemäßen Zerstäubers ähnelt den aus dem Stand der Technik bekannten Zerstäubungseinrichtungen. Durch die Vibrationseinrichtung, die vorzugsweise als PiezoVibrationseinrichtung ausgebildet ist, wird die Flüssigkeit in der Zerstäubungskammer in Schwingungen versetzt, so dass ein bei weitgehend konstanten Druckverhältnissen in der Zerstäubungskammer reproduzierbarer Austritt der Flüssigkeit durch die Vielzahl sehr kleiner Auslassöffnungen bewirkt wird. Die ausgetragene Flüssigkeit wird mittels der Pumpeinrichtung durch Flüssigkeit aus dem Flüssigkeitsspeicher ersetzt. Die Pumpeinrichtung selbst ist hierfür vorzugsweise als Mikropumpe ausgebildet. Eine solche Mikropumpe kann beispielsweise über eine mit einem Einlassventil und einem Auslassventil versehene Pumpkammer verfügen, welche mittels eines oder mehrerer Piezoaktoren zyklisch vergrößert und verkleinert wird.

Der Pump-Volumenstrom der Flüssigkeit, der durch die Pumpeinrichtung in Richtung der Zerstäubungskammer gefördert wird, ist zur Erzielung des homogenen Austragverhaltens größer als der durch die Auslassöffnungen abgegebene Austrag-Volumenstrom. Der Austragvolumenstrom hängt maßgeblich von der Bauweise der Zerstäubungseinrichtung sowie von der Ansteuerung der Vibrationseinrichtung durch die Steuereinrichtung ab. Der Pump-Volumenstrom hängt von der Gestaltung der Pumpeinrichtung selbst und deren Ansteuerung durch die Steuereinrichtung sowie von der Gestaltung der flüssigkeitsführenden Kanäle ab. Durch eine angemessene Auslegung der Zerstäubungseinrichtung, der Pumpeinrichtung und der Kanäle sowie insbesondere durch die Ansteuerung durch das Steuergerät kann der gewünschte den Austragvolumenstrom übersteigende Pumpvolumenstrom erzielt werden.

Von besonderem Vorteil ist es, wenn der Pump-Volumenstrom während der Zerstäubung um mindestens 20% über dem Austrag-Volumenstrom liegt. Besonders vorteilhaft ist es, wenn der Pump-Volumenstrom den Austrag-Volumenstrom noch deutlicher übersteigt. Besonders vorteilhaft ist es, wenn der Pump-Volumenstrom 40% oder mehr über dem Austrag-Volumenstrom liegt. Durch eine solche signifikant größere Förderung der Pumpeinrichtung gegenüber der Zerstäubung der Zerstäubungseinrichtung wird die Homogenität des Zerstäubungsvorgangs besonders gut gewährleistet.

Grundsätzlich ist es denkbar, dass die Steuereinrichtung dafür ausgebildet ist, die Pumpeinrichtung und die Vibrationseinrichtung in Reaktion auf ein Startsignal, beispielsweise in Reaktion auf eine Betätigung durch einen Benutzer, gleichzeitig zu starten. Als vorteilhaft wird es jedoch angesehen, wenn die Steuereinrichtung dafür ausgebildet ist, in einer Vorlaufphase vor Beginn des Zerstäubungsvorgangs zunächst die Pumpeinrichtung zu aktivieren und nach Ende der Vorlaufphase zusätzlich die Vibrationseinrichtung zu aktivieren. Die Vorlaufphase, die vorzugsweise mindestens 0,3 Sekunden dauert, insbesondere vorzugsweise mindestens 0,8 Sekunden, gewährleistet, dass die Zerstäubungskammer bereits vor Aktivierung der Vibrationseinrichtung vollständig mit Flüssigkeit gefüllt ist und sich ein durch die Pumpeinrichtung bewirkter Überdruck in der Zerstäubungskammer aufgebaut hat. Dies sind die idealen Randbedingungen, um anschließend die Vibrationseinrichtung zu aktivieren und damit den Zerstäubungsvorgang auszulösen.

Insbesondere damit während der Vorlaufphase kein Austreten der Flüssigkeit unter dem von der Pumpeinrichtung bewirkten Druck durch die Auslassöffnungen der Zerstäubungseinrichtung bewirkt wird, ist es von Vorteil, wenn die Pumpeinrichtung derart ausgebildet ist und/oder von der Steuereinrichtung derart angesteuert wird sowie die Auslassöffnungen der Zerstäubungseinrichtung derart ausgebildet sind, dass der sich in der Vorlaufphase in der Zerstäuberkammer einstellende Druck nicht ausreicht, um die Flüssigkeit durch die Auslassöffnungen hindurchzudrücken. Dies ist durch kleine Auslassöffnungen, durch die Formgebung der Auslassöffnungen und/oder durch einen geringen Druck in der Zerstäubungskammer zu erzielen. Der Druck ist so auszulegen, dass er erst zusammen mit den von der Vibrationseinrichtung bewirkten Schwingungen ausreicht, um die Flüssigkeit durch die Auslassöffnungen hindurchzupressen. Ein vergleichsweise geringer Druck in der Zerstäuberkammer lässt sich durch eine entsprechende Anpassung der Pumpeinrichtung bewerkstelligen. Vorteilhaft ist jedoch eine von der Pumpeinrichtung getrennte Drossel.

Als Flüssigkeitsspeicher kommt grundsätzlich jede Form eines Flüssigkeitsspeichers in Betracht. Im einfachsten Falle kann es sich beispielsweise um einen formstabilen Flüssigkeitsspeicher handeln, in den ein Steigrohr hineinragt, welches mit der Pumpeinrichtung verbunden ist. Insbesondere in Hinblick auf die Funktionstüchtigkeit des Zerstäubers bei variabler Ausrichtung wird es jedoch als vorteilhaft angesehen, wenn der Flüssigkeitsspeicher als formflexibler Flüssigkeitsbeutel ausgebildet ist, der sich hinsichtlich seines Innenvolumens der verbleibenden Flüssigkeitsmenge anpasst. Ein solcher formflexibler Flüssigkeitsbeutel bedarf keiner Belüftung, da sich der Druck der Flüssigkeit im Flüssigkeitsbeutel unabhängig vom verbleibenden Volumen nicht maßgeblich ändert, sondern stets dem Umgebungsdruck entspricht. Bis auf nachfolgend noch erwähnte Gasreste ist ein solcher Flüssigkeitsspeicher ausschließlich mit Flüssigkeit gefüllt, so dass stets gewährleistet ist, dass die Pumpeinrichtung Flüssigkeit ansaugt. Im Falle der vorliegenden Erfindung weist der Flüssigkeitsspeicher zwei Öffnungen auf, wobei einer mit dem Zulaufkanal und einer mit dem Rücklaufkanal verbunden ist. Die Verwendung eines Flüssigkeitsbeutels ist darüber hinaus aufgrund des dadurch erzielten sehr geringen Kontaminationsrisikos von Vorteil.

Unter anderem da jedoch auch bei einem solchen Flüssigkeitsbeutel nicht vollständig ausgeschlossen werden kann, dass bei der Befüllung auch Gas in den Flüssigkeitsbeutel gelangt und da es weiterhin möglich ist, dass Luft, die durch die Auslassöffnungen in die Zerstäubungskammer hineingelangt ist, durch den Rücklaufkanal ebenfalls in den Flüssigkeitsspeicher gelangt, ist es von Vorteil, wenn eine Blasenabscheideeinrichtung vorgesehen ist. Diese ist vorzugsweise im Zulaufkanal, insbesondere vorzugsweise zwischen der Pumpeinrichtung und der Zerstäubungskammer vorgesehen.

Dieser Blasenabscheideeinrichtung gewährleistet, dass die Flüssigkeit, die der Zerstäubungskammer zugeführt wird, zumindest weitgehend frei von Gasblasen ist. Die Anordnung der Blasenabscheideeinrichtung zwischen der Pumpeinrichtung und der Zerstäubungskammer führt darüber hinaus dazu, dass der pumpenausgangsseitig erzeugte Druck das Abscheiden von Gasblasen aus der Flüssigkeit in der Blasenabscheideeinrichtung begünstigt. Vorzugsweise weist die Blasenabscheideeinrichtung einen Kanalabschnitt auf, durch den hindurch die Flüssigkeit auf dem Weg zur Zerstäubungskammer fließt und der abschnittsweise durch eine Filterfläche begrenzt ist. Diese Filterfläche erlaubt bei dem durch die Pumpeeinrichtung erzeugten Druck das Austreten des Gases, während der pumpenausgangsseitige Druck nicht ausreicht, um die Flüssigkeit durch die Filterfläche hindurch zu drücken. Vorzugsweise ist die Filterfläche hydrophob ausgebildet, um den Kontakt zur Flüssigkeit soweit als möglich zu vermindern, so dass etwaige Gasblasen sich an der Filterfläche anlagern.

Eine besonders vorteilhafte Bauweise der Blasenabscheideeinrichtung sieht vor, dass der genannte Kanalabschnitt durch eine Nut in einem Basisbauteil gebildet wird, die durch die eben ausgebildete und auf diesem Basisbauteil aufgebrachte poröse Filterfläche verschlossen wird. Dies stellt eine baulich sehr einfache Gestaltung dar. Das Basisbauteil kann insbesondere als Kunststoffbauteil vorgesehen sein, in welches bereits bei der Herstellung die Nut eingebracht wird, die von der Flüssigkeit auf dem Weg zur Zerstäubungskammer durchquert werden muss. Diese Nut ist an ihrer offenen Seite durch die poröse Filterfläche verschlossen, welche zu diesem Zweck eben ausgebildet sein kann und daher sehr preisgünstig herzustellen ist. Die Verbindung zwischen dem Basisbauteil und der vorzugsweise als Membran ausgebildeten Filterfläche kann durch Kleben oder Schweißen hergestellt sein.

Als besonders vorteilhaft wird es angesehen, wenn der genannte Kanalabschnitt der Blasenabscheideeinrichtung mindestens einen Kurvenabschnitt oder Knickabschnitt aufweist. Dabei ist relevant, dass auch in diesem Abschnitt die poröse Filterfläche vorgesehen ist. Es hat sich herausgestellt, dass ein solcher Kurvenabschnitt das Austreten des Gases durch die poröse Filterfläche begünstigt, vermutlich da die Gasblasen sich mehrheitlich im Bereich der Innenkurve anordnen und dort mit verminderter Geschwindigkeit gefördert werden. Vorzugsweise sind mehrere verschieden relativ zueinander ausgerichtete Kurvenabschnitte oder Knickabschnitte vorgesehen, so dass auch bei variierender Ausrichtung des Zerstäubers ein Abscheiden der Gasbläschen erreicht wird. Des Weiteren ist durch eine solche Gestaltung des Kanals mit Kurven, die vorzugsweise geradlinige Abschnitte verbinden, auch bei einem vergleichsweise kleinen Bauteil ein langer Flüssigkeitspfad erzielbar, so dass Gasblasen über eine vergleichsweise lange Zeitdauer hinweg durch die poröse Filterfläche hindurch den Zulaufkanal verlassen können.

Wie oben bereits erwähnt, ist es besonders von Vorteil, wenn die im Flüssigkeitsspeicher enthaltene Flüssigkeit einen pharmazeutischen Wirkstoff enthält und/oder ein Kosmetikprodukt ist, insbesondere ein Selbstbräuner.

Ein erfindungsgemäßer Zerstäuber kann sowohl am Zulaufkanal als auch am Rücklaufkanal Durchflussmesser aufweisen, um durch die Bildung des Differenzwertes präzise bestimmen zu können, welche Flüssigkeitsmenge bei einem Austragvorgang zerstäubt wurde.

Der erfindungsgemäße Zerstäuber ist vorzugsweise als mobiles Gerät vorgesehen. Es weist zu diesem Zweck vorzugsweise eine Batterie oder einen Akkumulator auf. Eine besondere Gestaltung sieht vor, dass das Gerät eine werkzeuglos durch einen Benutzer austauschbare Verbrauchseinheit aufweist, die zumindest den Flüssigkeitsbeutel umfasst. Diese Verbrauchseinheit kann zusätzlich auch die Batterie aufweisen. Die vergleichsweise teuren Komponenten, wie insbesondere die elektronische Steuereinrichtung, die Pumpeinrichtung, die Blasenabscheideeinrichtung und die Zerstäubungseinrichtung sowie ggf. der Akkumulator, können Teil einer Basiseinheit sein, an der die Verbrauchseinheit ankoppelbar ist.

### Kurzbeschreibung der Zeichnungen

Weitere Aspekte und Vorteile der Erfindung ergeben sich außer aus den Ansprüchen auch aus der nachfolgenden Beschreibung eines bevorzugten Ausführungsbeispiels der Erfindung, welches anhand der Figuren erläutert wird. Dabei zeigen:
- Figur 1: einen erfindungsgemäßen Zerstäuber in einer Gesamtdarstellung,
- Figur 2: den Zerstäuber der Figur 1 nach Entfernen einer Gehäuseschale,
- Figur 3: den Zerstäuber der Figuren 1 und 2 in einer Teilexplosionsdarstellung und
- Figur 4: die Zerstäubungseinrichtung des Zerstäubers gemäß der Figuren 1 bis 3.

### Detaillierte Beschreibung des Ausführungsbeispiels

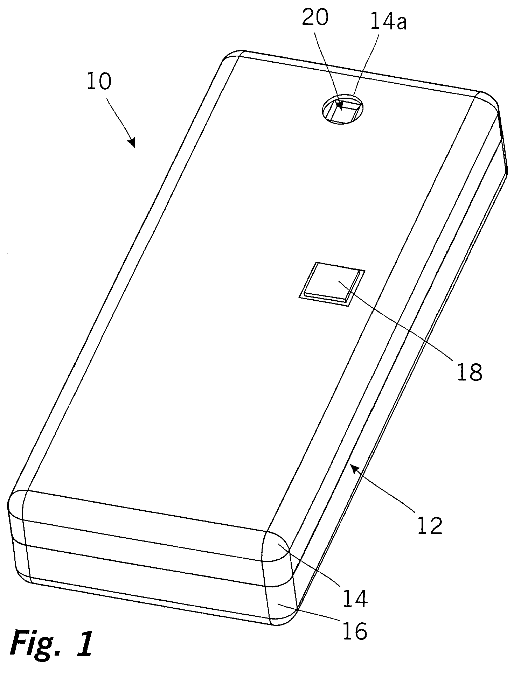

Figur 1 zeigt einen erfindungsgemäßen Zerstäuber 10 in einer Gesamtdarstellung. Der Zerstäuber 10 weist ein Gehäuse 12 in etwa von der Größe eines Mobiltelefons auf, welches über zwei Gehäuseschalen 14, 16 verfügt. In der oberen Gehäuseschale 14 ist eine Aussparung 14a vorgesehen, hinter der eine nachfolgend noch beschriebene Zerstäubungseinrichtung 20 angeordnet ist. Weiterhin ist an der von der Gehäuseschale 14 gebildeten Vorderseite ein Betätigungstaster 18 vorgesehen, mittels dessen der Zerstäuber 10 aktiviert werden kann.

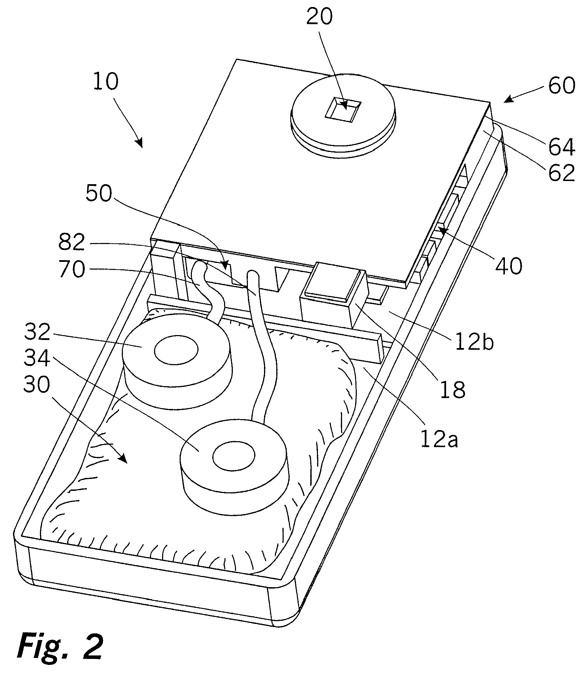

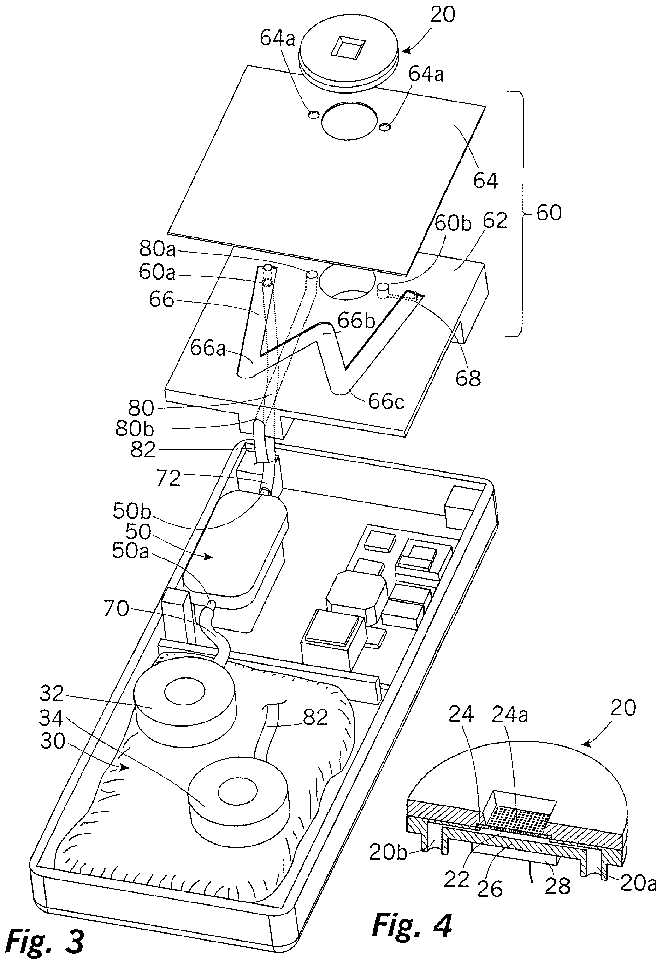

Die Figuren 2 und 3 zeigen den Zerstäuber 10 im geöffneten Zustand, also nach Entfernen der oberen Gehäuseschale 14. Dabei sind in Figur 3 einige Komponenten in einer aus dem Gehäuse 12 entfernten Position dargestellt, um alle maßgeblichen Komponenten erkennen zu können.

Innerhalb des Gehäuses 12 sind zwei getrennte Bereiche 12a, 12b vorgesehen. Der Bereich 12a beinhaltet einen Flüssigkeitsspeicher 30, der in Form eines Flüssigkeitsbeutels 30 vorgesehen ist. Am Flüssigkeitsbeutel 30 sind zwei in den Darstellungen verdeckte Öffnungen vorgesehen, auf denen jeweils eine Kopplungseinrichtung 32, 34 zum Anschließen jeweils einer Schlauchleitung vorgesehen ist.

Im Bereich 12b des Gehäuses 12 sind der bereits genannte Betätigungstaster 18 sowie die genannte Zerstäubungseinrichtung 20 angeordnet. Weiterhin sind hier eine Steuereinrichtung 40 und ein nicht dargestellter Akkumulator als Energiespeicher vorgesehen. Der Bereich 12b beinhaltet darüber hinaus eine Piezopumpeinrichtung 50 sowie eine Blasenabscheideeinrichtung 60.

Der Flüssigkeitsbeutel 30 ist folgendermaßen mit der Zerstäubungseinrichtung 20 verbunden. Über einen Schlauchabschnitt 70 ist die Kopplungseinrichtung 32 mit der Eingangsseite 50a der Pumpeinrichtung 50 verbunden. An der Ausgangsseite 50b der Pumpeinrichtung 50 ist ein weiterer Schlauchabschnitt 72 vorgesehen, der mit einem Einlass 60a der Blasenabscheideeinrichtung 60 verbunden ist. Dieser Einlass 60a mündet in einen Blasenabscheidekanal 66, der als Nut in ein Basisbauteil 62 der Blasenabscheideeinrichtung 60 eingebracht ist. In der in Figur 2 verdeutlichten Weise ist das Basisbauteil 62 im zusammengesetzten Zustand des Zerstäubers 10 durch eine Membran 64, insbesondere eine Membran auf einer Akryl-Copolymer-Basis mit hydrophober Deckschicht, flüssigkeitsdicht verschlossen, die mit dem Basisbauteil 62 im Randbereich der Nut verklebt oder verschweißt sein kann. Diese Membran 64 ist als poröse Membran ausgebildet, die dem Zweck dient, Gasblasen aus der Flüssigkeit, die den Kanal 66 entlangströmt, entweichen zu lassen. Am gegenüberliegenden Ende des Kanals 66 ist eine Drossel in Form eines Drosselkanalabschnitts 68 vorgesehen, dessen Querschnittsfläche wesentlich geringer als die Querschnittsfläche des vorherigen Kanalabschnitts 66 ist. Dieser Drosselkanalabschnitt 68 mündet in einen Auslass 60b der Blasenabschneideeinrichtung 60. An diesen Auslass 60b des Blasenabscheiders 60 ist unmittelbar die in Figur 4 vergrößert dargestellte Zerstäubungseinrichtung 20 vorgesehen, wobei zur Schaffung dieser unmittelbaren Verbindung in der Membran 64 Durchbrechungen 64a vorgesehen sind.

Die Zerstäubungseinrichtung 20 verfügt über eine Zerstäubungskammer 22, die zwischen einem Flüssigkeitseinlass 20a und einem Flüssigkeitsauslass 20b angeordnet ist. Dieser Zerstäubungskammer 22 ist auf ihrer in Richtung der Durchbrechung 14a der oberen Gehäuseschale 14 weisenden Seite von einer Lochplatte 24 mit einer Vielzahl von Auslassöffnungen 24a begrenzt. Auf der der Lochplatte 24 abgewandten Seite der Zerstäubungskammer 22 ist eine Wandung 26 vorgesehen, an der eine Piezovibrationseinrichtung 28 befestigt ist, welche über die näher dargestellte Kabel mit der Steuereinrichtung 40 verbunden ist. Im Basisbauteil 62 und in der Membran 64 sind Durchbrechungen zur Aufnahme und Verkabelung der Piezovibrationseinrichtung 28 vorgesehen.

Für die überschüssige Flüssigkeit aus der Zerstäubungskammer 22 ist der Flüssigkeitsauslass 20b vorgesehen. Dieser ist im zusammengesetzten Zustand mit dem Einlass 80a eines Flüssigkeitskanals 80 verbunden, der das Basisbauteil 62 der Blasenabscheideeinrichtung 60 bis zu seinem Auslass 80b durchquert, ohne dass diesem Kanalabschnitt 80 eine Blasenabscheidefunktion zugeordnet wäre. An der Auslassseite 80b schließt sich eine Schlauchleitung 82 an, die zur oben beschriebenen zweiten Kopplungseinrichtung 34 des Flüssigkeitsbeutels 30 führt.

Über die Kanalabschnitte 70, 72, 66 ist der Flüssigkeitsbeutel 30 somit mit der Zerstäubungseinrichtung 20 verbunden. Dies stellt den Zulaufkanal dar. Der Rücklaufkanal wird durch die Kanalabschnitte 80 und 82 gebildet.

Der Betrieb des Zerstäubers 10 wird nachfolgend erläutert.

Zum Austrag der Flüssigkeit aus dem Flüssigkeitsspeicher 30 betätigt ein Benutzer den Betätigungstaster 18. Dies wird durch die elektronische Steuereinrichtung 40 registriert. Die elektronische Steuereinrichtung 40 aktiviert zur Vorbereitung eines nachfolgenden Zerstäubungsvorgangs zunächst isoliert die piezobetriebene Mikropumpeinrichtung 50. Hierdurch wird Flüssigkeit durch den Schlauchabschnitt 70 aus dem Flüssigkeitsspeicher 30 angesogen und durch den Schlauchabschnitt 72 weiter zur Blasenabscheideeinrichtung 60 gefördert. Hier gelangt die Flüssigkeit in den Kanalabschnitt 66. Aufgrund des an der Ausgangsseite 50b der Pumpeinrichtung 50 aufgebauten Pumpdrucks in Verbindung mit der Drosselwirkung des Drosselkanalabschnitts 68 wird innerhalb des Kanalabschnitts 66 ein vergleichsweise hoher Druck aufgebaut. Der Überdruck im Kanalabschnitt 66gegenüber einer Umgebung liegt vorzugsweise bei 100 mbar - 500 mbar. Dieser reicht aus, um Gasbläschen aus der angesogenen Flüssigkeit durch die poröse Membran 64 hindurch abzugeben, wobei das Gas dann durch Spalte des Gehäuses 12 entweichen kann. Die durch die Mikropumpeinrichtung 50 geförderte Flüssigkeit kann jedoch bei dem genannten Überdruck nicht durch die poröse Membran 64 entweichen. Es wird bewirkt, dass die in den Drosselkanal 68 eindringende Flüssigkeit weitgehend gasblasenfrei ist. Durch die W-förmige Gestaltung des Kanalabschnitts 66, insbesondere durch drei Kurve 66a, 66b, 66c wird der Blasenabscheidevorgang begünstigt, da sich die Blasen im Bereich dieser Kurven 66a, 66b, 66c an der Innenkurve sammeln und dort vergleichsweise langsam weitergefördert werden. Hierdurch ist ausreichend Zeit für die Gasblasen gegeben, um durch die poröse Membran 64 hindurch zu entweichen. Der Drosselkanalabschnitt 68, der unmittelbar der Zerstäubungseinrichtung 20 vorgeschaltet ist, führt zu einer signifikanten Druckverringerung der Flüssigkeit. Die von Gasblasen weitgehend befreite Flüssigkeit gelangt daher mit einem reduzierten Druck in die Zerstäubungskammer 22. Da die Vibrationseinrichtung 28 zu diesem Zeitpunkt noch nicht aktiviert ist, strömt die Flüssigkeit von der Eingangsseite 20a der Zerstäubungseinrichtung 20 unmittelbar zur Ausgangsseite 20b der Zerstäubungseinrichtung 20. Der durch die Drossel 68 auf einen Überdruck zwischen 0 mbar und 30 mbar, vorzugsweise zwischen 2 mbar und 10 mbar, reduzierte Flüssigkeitsdruck reicht zunächst nicht aus, um die Flüssigkeit durch die Auslassöffnungen 24a nach außen zu pressen. Es kommt daher nicht zu einem Nässen an den Auslassöffnungen 24a. Stattdessen gelangt die gesamte geförderte Flüssigkeit in der Vorlaufphase aufgrund des geringen Strömungswiderstandes in den Kanalabschnitten 80, 82 zurück in den Flüssigkeitsspeicher. In der Vorlaufphase, in der lediglich die Pumpeinrichtung 50, jedoch nicht die Vibrationseinrichtung 28, aktiviert ist, strömt der vollständige Pump-Volumenstrom somit im Kreis. Gegebenenfalls vor Betätigen des Betätigungstasters 18 noch in der Zerstäubungskammer 22 befindliche Luft wird bei Einströmen der Flüssigkeit in die Zerstäubungskammer entweder durch die Auslassöffnungen 24a hindurch aus der Zerstäubungskammer 22 herausgepresst, oder sie gelangt über die Kanalabschnitte 80, 82 bis in den Flüssigkeitsbeutel 30 und wird beim nächsten Ansaugen in der Blasenabscheideeinrichtung 60 von der Flüssigkeit getrennt.

Nach Ende der Vorlaufphase, vorzugsweise nach etwa einer Sekunde, wird von der Steuereinrichtung 40 auch die Vibrationseinrichtung 28 aktiviert. Hierdurch wird bewirkt, dass die in der Zerstäubungskammer 22 enthaltene Flüssigkeit in Schwingungen versetzt wird, was ein Austreten der Flüssigkeit in Form kleinster Tröpfchen durch die Auslassöffnungen 24a zur Folge hat. Es wird somit ein feiner Flüssigkeitsnebel durch die Öffnung 14a in die obere Gehäuseschale 14 abgegeben, wobei dieser Flüssigkeitsnebel beispielsweise als Medikament inhaliert werden kann oder bei einer entsprechenden Flüssigkeit als kosmetischer Niederschlag auf die Haut abgegeben werden kann.

Ab der Inbetriebnahme der Vibrationseinrichtung 28 wird ein Teil, vorzugsweise die Hälfte oder ein überwiegender Teil des Pumpvolumenstroms, der von der Pumpeinrichtung 50 in Richtung der Zerstäubungseinrichtung 20 gefördert wird, durch die Auslassöffnungen 24a abgegeben. Dennoch verbleibt ein relevanter Anteil der von der Pumpeinrichtung 50 geförderten Flüssigkeit, der durch die Kanalabschnitte 80, 82 hindurch zurück in den Flüssigkeitsbeutel 30 gefördert wird. Hierdurch ist gewährleistet, dass unbeabsichtigt in die Zerstäubungskammer 22 eingedrungene Luft zuverlässig aus dieser abgeführt wird. Gleichzeitig führt der gegenüber dem Austrag-Volumenstrom größere PumpVolumenstrom dazu, dass zuverlässig stets eine ausreichende Menge Flüssigkeit in der Zerstäubungskammer 22 vorhanden ist.

## Patentansprüche

1. Zerstäuber (10) zur Zerstäubung von Flüssigkeiten mit
- einem Flüssigkeitsspeicher (30),
- einer Zerstäubungseinrichtung (20) mit einer Zerstäubungskammer (22), die einerseits durch eine Vielzahl von Auslassöffnungen (24a) mit einer Umgebungsatmosphäre verbunden ist, und andererseits mit einer Vibrationseinrichtung (28), mittels derer eine auf der den Auslassöffnungen (24a) abgewandten Seite der Zerstäubungskammer (22) angeordnete Wandung und hierdurch zum Zwecke der Zerstäubung Flüssigkeit in der Zerstäubungskammer (22) in Schwingungen versetzbar sind, wobei von der Zerstäubungseinrichtung (20) durch die Auslassöffnungen (24a) ein AustragVolumenstrom abgegeben wird,
- einem Zulaufkanal (70, 72, 66, 68), durch den der Flüssigkeitsspeicher (30) mit der Zerstäubungseinrichtung (20) verbunden ist,
- einer Pumpeinrichtung (50), die im Zulaufkanal (70, 72, 66, 68) zum Zwecke der Förderung der Flüssigkeit vom Flüssigkeitsspeicher (30) zur Zerstäubungskammer (22) vorgesehen ist,
- einer elektronischen Steuereinrichtung (40) zur Steuerung der Pumpeinrichtung (50) und der Vibrationseinrichtung (28), und
- einem vom Zulaufkanal (70, 72, 66, 68) getrennten Rücklaufkanal (80, 82) zum Abfluss von Flüssigkeit aus der Zerstäubungskammer (22) in den Flüssigkeitsspeicher (30),
- im Zulaufkanal (70, 72, 66, 68) eine Blasenabscheidungseinrichtung (60) vorgesehen ist,
**dadurch gekennzeichnet, dass**
der Zerstäuber insbesondere für Zerstäubungsvorgange bei variabler Ausrichtung des Zerstäubers ausgebildet ist, wobei
- der Flüssigkeitsspeicher (30) als formflexibler Flüssigkeitsbeutel (30) ausgebildet ist,
- die Pumpeinrichtung (50) durch die Steuereinrichtung (40) zum Zwecke der kontinuierlichen Förderung der Flüssigkeit vom Flüssigkeitsspeicher (30) zur Zerstäubungskammer (22) ansteuerbar ist und
- die Zerstäubungseinrichtung (20) und die Pumpeinrichtung (50) derart ausgebildet sind und/oder derart von der Steuereinrichtung (40) ansteuerbar sind, dass während der Zerstäubung der durch die Pumpeinrichtung (50) in die Zerstäubungskammer (22) kontinuierlich geförderte PumpVolumenstrom der Flüssigkeit größer ist als der von der Zerstäubungseinrichtung (20) durch die Auslassöffnungen (24a) abgegebene Austrag-Volumenstrom, wobei eine überschüssige Flüssigkeit über den Rücklaufkanal (80, 82) von der Zerstäubungskammer (22) in den Flüssigkeitsspeicher (30) gefördert wird.

2. Zerstäuber (10) nach Anspruch 1,
**dadurch gekennzeichnet, dass**
der Pump-Volumenstrom während der Zerstäubung um mindestens 20% über dem Austrag-Volumenstrom liegt.

3. Zerstäuber (10) nach einem der vorstehenden Ansprüche,
**dadurch gekennzeichnet, dass**
die Steuereinrichtung (40) dafür ausgebildet ist, in einer Vorlaufphase vor Beginn des Austragvorgangs zunächst nur die Pumpeinrichtung (50) zu aktivieren und nach Ende der Vorlaufphase zusätzlich die Vibrationseinrichtung (28) zu aktivieren.

4. Zerstäuber (10) nach Anspruch 3,
**dadurch gekennzeichnet, dass**
die Pumpeinrichtung (50) derart ausgebildet ist und/oder von der Steuereinrichtung (40) angesteuert wird und die Auslassöffnungen (24a) der Zerstäubungseinrichtung (20) derart ausgebildet sind, dass der sich in der Vorlaufphase in der Zerstäuberkammer (22) einstellende Druck nicht ausreicht, um Flüssigkeit durch die Auslassöffnungen (24a) hindurchzudrücken.

5. Zerstäuber (10) nach einem der vorstehenden Ansprüche,
**dadurch gekennzeichnet, dass**
die Blasenabscheideeinrichtung (60) einen Kanalabschnitt (66) aufweist, durch den die Flüssigkeit auf dem Weg zur Zerstäubungskammer (22) fließt und der abschnittsweise durch eine Filterfläche (64) begrenzt ist.

6. Zerstäuber (10) nach Anspruch 5,
**dadurch gekennzeichnet, dass**
die Pumpeinrichtung (50) derart ausgebildet und/oder von der Steuereinrichtung (40) angesteuert wird und die Blasenabscheideeinrichtung (60) derart ausgebildet ist, dass der sich in der Blasenabscheideeinrichtung (60) einstellende Druck ausreicht, um Gas durch die poröse Filterfläche (64) hindurchzudrücken, und nicht ausreicht, um Flüssigkeit durch die poröse Filterfläche (64) hindurchzudrücken.

7. Zerstäuber (10) nach einem der vorstehenden Ansprüche,
**dadurch gekennzeichnet, dass**
der Kanalabschnitt (66) der Blasenabscheideeinrichtung (60)
- durch eine Nut in einem ersten Basisbauteil (62) gebildet wird, die durch die eben ausgebildete und auf diesem Bauteil aufgebrachte poröse Filterfläche (64) verschlossen wird, und/oder
- mindestens zwei geradlinige Abschnitte aufweist, die durch mindestens einen Kurvenabschnitt (66a, 66b, 66c) miteinander verbunden sind.

8. Zerstäuber (10) nach einem der vorstehenden Ansprüche,
**dadurch gekennzeichnet, dass**
die im Flüssigkeitsspeicher ((30) enthaltene Flüssigkeit einen pharmazeutischen Wirkstoff enthält und/oder ein Kosmetikprodukt ist.

9. Zerstäuber (10) nach Anspruch 8,
**dadurch gekennzeichnet, dass**
das Kosmetikprodukt eine Selbstbräunungsflüssigkeit ist.

## Claims

1. Atomizer (10) for atomizing liquids, with
- a liquid storage receptacle (30),
- an atomizing device (20) with an atomizing chamber (22), which on the one hand is connected to an ambient atmosphere via a multiplicity of outlet openings (24a), and on the other hand with a vibrator (28), by means of which a wall arranged on the side of the atomizing chamber (22) directed away from the outlet openings (24a) can be caused to vibrate, and in this way liquid in the atomizing chamber (22) is caused to vibrate for the purpose of atomization, wherein a discharged volumetric flow rate is delivered from the atomizing device (20) through the outlet openings (24a),
- an intake channel (70, 72, 66, 68), via which the liquid storage receptacle (30) is connected to the atomizing device (20),
- a pump device (50), which is provided in the intake channel (70, 72, 66, 68) for the purpose of conveying the liquid from the liquid storage receptacle (30) to the atomizing chamber (22),
- an electronic control device (40) for controlling the pump device (50) and the vibrator (28), and
- a return channel (80, 82), which is separate from the intake channel (70, 72, 66, 68) and serves to drain liquid from the atomizing chamber (22) into the liquid storage receptacle (30),
- a bubble separator (60) is provided in the intake channel (70, 72, 66, 68),
**characterized in that**
the atomizer is designed in particular for atomizing operations with variable orientation of the atomizer, wherein
- the liquid storage receptacle (30) is designed as a dimensionally flexible liquid-containing pouch (30),
- the pump device (50) can be controlled by the control device (40) for the purpose of continuously conveying the liquid from the liquid storage receptacle (30) to the atomizing chamber (22), and
- the atomizing device (20) and the pump device (50) are designed in such a way and/or can be controlled by the control device (40) in such a way that, during the atomization, the pumped volumetric flow rate of the liquid conveyed continuously by the pump device (50) into the atomizing chamber (22) is greater than the discharged volumetric flow rate delivered from the atomizing device (20) through the outlet openings (24a), wherein excess liquid is conveyed from the atomizing chamber (22) through the return channel (80, 82) to the liquid storage receptacle (30).

2. Atomizer (10) according to Claim 1, **characterized in that** the pumped volumetric flow rate during the atomization is at least 20% above the discharged volumetric flow rate.

3. Atomizer (10) according to either of the preceding claims, **characterized in that** the control device (40) is designed to initially activate only the pump device (50) in a preparatory phase before the start of the discharging operation, and to additionally activate the vibrator (28) after completion of the preparatory phase.

4. Atomizer (10) according to Claim 3, **characterized in that** the pump device (50) is designed in such a way and/or is controlled by the control device (40) in such a way, and the outlet openings (24a) of the atomizing device (20) are designed in such a way, that the pressure set in the atomizing chamber (22) in the preparatory phase is not sufficient to force liquid through the outlet openings (24a).

5. Atomizer (10) according to one of the preceding claims, **characterized in that** the bubble separator (60) has a channel portion (66), through which the liquid flows on its way to the atomizing chamber (22) and which is delimited in parts by a filter surface (64).

6. Atomizer (10) according to Claim 5, **characterized in that** the pump device (50) is designed in such a way and/or is controlled by the control device (40) in such a way, and the bubble separator (60) is designed in such a way, that the pressure set in the bubble separator (60) is sufficient to force gas through the porous filter surface (64) but is not sufficient to force liquid through said porous filter surface (64).

7. Atomizer (10) according to one of the preceding claims, **characterized in that** the channel portion (66) of the bubble separator (60)
- is formed by a groove in a first base component (62), which groove is closed by the flat porous filter surface (64) mounted on this component, and/or
- has at least two straight portions, which are connected to each other by at least one curved portion (66a, 66b, 66c).

8. Atomizer (10) according to one of the preceding claims, **characterized in that** the liquid in the liquid storage receptacle (30) contains a pharmaceutical active substance and/or is a cosmetic product.

9. Atomizer (10) according to Claim 8, **characterized in that** the cosmetic product is a self-tanning lotion.

## Revendications

1. Pulvérisateur (10) pour la pulvérisation de liquides, comprenant
- un réservoir de liquide (30),
- un dispositif de pulvérisation (20) comprenant une chambre de pulvérisation (22), qui est reliée d'un côté par une pluralité d'ouvertures de sortie (24a) avec une atmosphère ambiante et d'un autre côté avec un dispositif de vibration (28), au moyen duquel une paroi agencée sur la face opposée aux ouvertures de sortie (24a) de la chambre de pulvérisation (22) et ainsi pour la pulvérisation un liquide dans la chambre de pulvérisation (22) sont mis en vibration, un courant volumique de sortie étant déchargé du dispositif de pulvérisation (20) par les ouvertures de sortie (24a),
- un canal d'alimentation (70, 72, 66, 68), par lequel le réservoir de liquide (30) est relié avec le dispositif de pulvérisation (20),
- un dispositif de pompage (50), qui est prévu dans le canal d'alimentation (70, 72, 66, 68) pour le transport du liquide depuis le réservoir de liquide (30) vers la chambre de pulvérisation (22),
- un dispositif de commande électronique (40) pour la commande du dispositif de pompage (50) et du dispositif de vibration (28), et
- un canal de reflux (80, 82) séparé du canal d'alimentation (70, 72, 66, 68), pour l'évacuation de liquide depuis la chambre de pulvérisation (22) dans le réservoir de liquide (30),
- un dispositif de séparation des bulles (60) étant prévu dans le canal d'alimentation (70, 72, 66, 68), **caractérisé en ce que** le pulvérisateur est configuré notamment pour les processus de pulvérisation à orientation variable du pulvérisateur,
- le réservoir de liquide (30) étant configuré sous la forme d'un sac à liquide de forme flexible (30),
- le dispositif de pompage (50) pouvant être commandé par le dispositif de commande (40) pour le transport continu du liquide depuis le réservoir de liquide (30) vers la chambre de pulvérisation (22), et
- le dispositif de pulvérisation (20) et le dispositif de pompage (50) étant configurés et/ou pouvant être commandés par le dispositif de commande (40) de sorte que, pendant la pulvérisation, le courant volumique de liquide pompé transporté en continu par le dispositif de pompage (50) dans la chambre de pulvérisation (22) soit supérieur au courant volumique de sortie déchargé par les ouvertures de sortie (24a) par le dispositif de pulvérisation (20), un excès de liquide étant transporté par le canal de reflux (80, 82) depuis la chambre de pulvérisation (22) dans le réservoir de liquide (30).

2. Pulvérisateur (10) selon la revendication 1, **caractérisé en ce que** le courant volumique pompé pendant la pulvérisation est supérieur d'au moins 20 % au courant volumique de sortie.

3. Pulvérisateur (10) selon l'une quelconque des revendications précédentes, **caractérisé en ce que** le dispositif de commande (40) est configuré pour n'activer tout d'abord que le dispositif de pompage (50) pendant une phase préliminaire avant le début du processus de déchargement, et pour activer en outre le dispositif de vibration (28) après la fin de la phase préliminaire.

4. Pulvérisateur (10) selon la revendication 3, **caractérisé en ce que** le dispositif de pompage (50) est configuré et/ou commandé par le dispositif de commande (40) et les ouvertures de sortie (24a) du dispositif de pulvérisation (20) sont configurées pour que la pression qui s'ajuste dans la chambre de pulvérisation (22) pendant la phase préliminaire ne soit pas suffisante pour pousser le liquide au travers des ouvertures de sortie (24a).

5. Pulvérisateur (10) selon l'une quelconque des revendications précédentes, **caractérisé en ce que** le dispositif de séparation des bulles (60) comprend une section de canal (66) dans laquelle le liquide s'écoule sur sa trajectoire vers la chambre de pulvérisation (22) et qui est délimitée par sections par une surface de filtration (64).

6. Pulvérisateur (10) selon la revendication 5, **caractérisé en ce que** le dispositif de pompage (50) est configuré et/ou commandé par le dispositif de commande (40) et le dispositif de séparation des bulles (60) est configuré pour que la pression qui s'ajuste dans le dispositif de séparation des bulles (60) soit suffisante pour pousser le gaz au travers de la surface de filtration poreuse (64) et ne soit pas suffisante pour pousser le liquide au travers de la surface de filtration poreuse (64).

7. Pulvérisateur (10) selon l'une quelconque des revendications précédentes, **caractérisé en ce que** la section de canal (66) du dispositif de séparation des bulles (60)
- est formée par une rainure dans un premier composant de base (62), qui est fermée par la surface de filtration poreuse (64) configurée sous forme plate et disposée sur ce composant, et/ou
- comprend au moins deux sections rectilignes qui sont reliées l'une avec l'autre par au moins une section incurvée (66a, 66b, 66c).

8. Pulvérisateur (10) selon l'une quelconque des revendications précédentes, **caractérisé en ce que** le liquide contenu dans le réservoir de liquide (30) contient un agent actif pharmaceutique et/ou est un produit cosmétique.

9. Pulvérisateur (10) selon la revendication 8, **caractérisé en ce que** le produit cosmétique est un liquide autobronzant.
